(19) Europäisches Patentamt
European Patent Office
Office européen des brevets

(11) Veröffentlichungsnummer: **0 255 650 B1**

(12) # EUROPÄISCHE PATENTSCHRIFT

(45) Veröffentlichungstag der Patentschrift: **10.04.91**

(51) Int. Cl.5: **C07C 13/465**, C07C 2/68

(21) Anmeldenummer: **87110537.5**

(22) Anmeldetag: **21.07.87**

(54) **Verfahren zur Herstellung von Indanen.**

(30) Priorität: **02.08.86 DE 3626227**

(43) Veröffentlichungstag der Anmeldung:
**10.02.88 Patentblatt 88/06**

(45) Bekanntmachung des Hinweises auf die Patenterteilung:
**10.04.91 Patentblatt 91/15**

(84) Benannte Vertragsstaaten:
**BE CH DE FR GB IT LI NL**

(56) Entgegenhaltungen:

**JOURNAL OF CHEMICAL RESEARCH (S), 1979, Seiten 184,185, Paris, FR; F. MARCUZZI et al.: "Synthesis and configuration of 1,2,3-triphenyl- and 1,3-diphenyl-2-methyl-indanes"**

(73) Patentinhaber: **BASF Aktiengesellschaft**
**Carl-Bosch-Strasse 38**
**W-6700 Ludwigshafen(DE)**

(72) Erfinder: **Henkes, Erhard, Dr.**
**Dr.-Kurt-Schumacher-Strasse 1**
**W-6141 Einhausen(DE)**
Erfinder: **Halbritter, Klaus, Dr.**
**Schwarzwaldstrasse 19**
**W-6800 Mannheim 1(DE)**
Erfinder: **Mayr, Herbert, Prof. Dr.**
**Sandsteinstrasse 7**
**W-2401 Gross Groenau(DE)**
Erfinder: **Striepe, Wilhelm, Dr.**
**Bismarckstrasse 5**
**W-8670 Hof(DE)**
Erfinder: **Pock, Rudolf, Dr.**
**Paul-Gossen-Strasse 34**
**W-8520 Erlangen(DE)**

**EP 0 255 650 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Indanen durch Umsetzung von gegebenenfalls substituierten Benzylhalogeniden mit Olefinen in Gegenwart einer Lewis-Säure als Katalysator.

Die Umsetzung von substituierten Benzylhalogeniden mit Olefinen wie Propen, Isobuten und 1-Methylstyrol in Gegenwart von Zinkchlorid bzw. Zinkchloridetherat in Dichlormethan als Lösungsmittel wird in J. Org. Chem., Bd. 48, 1159-1165 (1983) beschrieben. Dabei wird sowohl bei tiefen Temperaturen von -78°C als auch bei Temperaturen von 0 bis +80°C in der Regel das 1 : 1-Additionsprodukt erhalten. (s. auch J. Org. Chem. Bd. 29, 2685-2687, 1964). Die nachfolgende Ringschlußreaktion zum Indan wird lediglich bei Umsetzung von 1-Chlor-1-phenylethan mit 1-Methylstyrol bei +50°C und bei Umsetzung von Tritylchlorid mit Isobuten bei 0°C in Ausbeuten von 80 bzw. 21 % beobachtet. Aus der Dissertation von W. Striepe, Erlangen, 1984, Seiten 28, 29 und 119 geht hervor, daß Indane erhalten werden können, wenn man p-Methylcumylchlorid mit alkylsubstituierten Olefinen zunächst bei tiefer Temperatur in Gegenwart von Zinkchloridetherat umsetzt und dann das Reaktionsgemisch innerhalb von 10 h auf Raumtemperatur erwärmen läßt. Dieses Verfahren ist jedoch aufgrund der tiefen Temperatur und einer geringen Raum-Zeit-Ausbeute technisch wenig brauchbar.

Aus J. Org. Chem., Bd. 44, 3022-3028 (1979), und J. Chem. Res. (S) 1979, S. 184 u. 185 ist bekannt, daß die Umsetzung von Phenyl- und Diphenylalkylchloriden mit phenylsubsituierten Alkenen wie Phenylpropen und Stilben in Gegenwart von Zinkchlorid in Dichlormethan als Lösungsmittel und bei Temperaturen von 20 bis 40°C in Ausbeuten von 70 bis 84 % zu den entsprechenden Indanen führen. Dagegen fällt mit Styrol als Olefin das 1 : 1-Additionsprodukt als Hauptprodukt an.

Nachteilig an den beschriebenen Verfahren ist die zum Teil geringe bis mäßige Ausbeute an Indanen, die geringe Anwendungsbreite hinsichtlich der Ausgangskomponenten bei für technische Prozesse interessanten Temperaturbereichen, sowie die Notwendigkeit, die Umsetzung in Gegenwart von Lösungsmitteln, insbesondere von toxikologisch nicht unbedenklichen halogenierten Kohlenwasserstoffen durchzuführen.

Der Erfindung lag daher die Aufgabe zugrunde, ein Verfahren zu finden, nach dem Indane auf technisch einfache Weise durch Lewissäure-katalysierte Umsetzung von gegebenenfalls substituierten Benzylhalogeniden mit Olefinen in hohen Ausbeuten und Raum-Zeit-Ausbeuten anfallen und bei dem Olefine eingesetzt werden können, die nicht durch Arylreste substituiert sind.

Demgemäß wurde ein Verfahren zur Herstellung von Indanen durch Umsetzung von gegebenenfalls substituierten Benzylhalogeniden mit Olefinen in Gegenwart einer Lewis-Säure als Katalysator gefunden, das dadurch gekennzeichnet ist, daß man Benzylhalogenide der Formel I

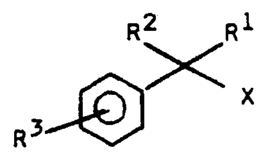

I,

in der die Reste $R^1$, $R^2$ und $R^3$ Wasserstoff oder einen Alkyl-, Alkoxy-, Cycloalkyl-, Aryl-, Aryloxy- oder Aralkylrest bedeuten, $R^3$ darüber hinaus für Halogen stehen kann oder ein ortho-kondensiertes Ringsystem bildet und X Halogen bedeutet, mit Olefinen der Formel II

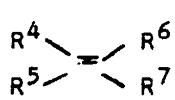

II,

in der die Reste $R^4$ bis $R^7$ Wasserstoff oder einen in alpha-Stellung unverzweigten Alkylrest darstellen, in Gegenwart katalytischer Mengen an Titantetrachlorid umsetzt.

Nach dem erfindungsgemäßen Verfahren werden Benzylhalogenide I, z.B. ggf. substituierte Benzylfluoride, -chloride oder -bromide umgesetzt. Da Benzylchloride am einfachsten zugänglich sind, werden sie bevorzugt verwendet. Die Herstellung von substituierten Benzyyfluoriden oder -bromiden ist z.B. in J. Org. Chem. 29, 2685-87 (1964) beschrieben.

Als Reste $R^1$ und $R^2$ kommen neben Wasserstoff verzweigte oder unverzweigte Alkylreste, z.B. $C_1$- bis $C_{12}$-, insbesondere $C_1$- bis $C_8$-, vorzugsweise $C_1$-bis $C_4$-Alkylreste, Alkoxyreste, z.B. $C_1$- bis $C_8$-, vorzugs-

weise $C_1$- bis $C_4$-Alkoxyreste, $C_5$- oder $C_6$-Cycloalkylreste, Arylreste, z.B. Phenyl- oder inerte Substituenten tragende Phenylreste wie halogen-, alkyl- oder alkoxysubstituierte Phenylreste, Aryloxyreste z.B. der Phenoxyrest sowie Aralkylreste, z.B. $C_6$- bis $C_{12}$-Aralkylreste in Betracht. Beispielsweise seien der Methyl-, Ethyl-, Propyl-, Isopropyl-, Butyl-, tert.-Butyl-, Pentyl-, Hexyl-, Octyl-, Methoxy-, Ethoxy-, Cyclopentyl-, Cyclohexyl-, Phenyl-, p-Chlor-phenyl-, Tolyl-, Phenoxy-, Phenylethyl- oder Benzylrest aufgeführt.

Der Rest $R^3$ hat die für $R^1$ und $R^2$ genannte Bedeutung und kann darüber hinaus für Halogen, z.B. Fluor, Chlor oder Brom stehen oder ein ortho-kondensiertes Ringsystem, z.B. zusammem mit dem Phenylkern ein Naphthylsystem, bilden.

In den Olefinen II stehen die Reste $R^4$ bis $R^7$ für Wasserstoff oder einen Alkylrest, der in alpha-Stellung unverzweigt ist, ansonsten aber verzweigt sein kann. Die Alkylreste können z.B. 1 bis 12, insbesondere 1 bis 8, vorzugsweise 1 bis 4 Kohlenstoffatome enthalten, z.B. sei der Methyl-, Ethyl-, Propyl-, Butyl-, 2-Methyl-butyl-, 3,3-Dimethylbutyl-, Pentyl-, Hexyl- oder Octylrest aufgeführt. Vorzugsweise können di-, tri- und tetra-substituierte Olefine umgesetzt werden, z.B. Propen, But-1-en, Isobuten, Pent-1-en, Hex-1-en, But-2-en, 2-Methylbut-2-en, 3-Methylpent-2-en, 3,5-Dimetnylhex-2-en, Tetramethylethylen.

Im allgemeinen können 1,5 bis 4, vorteilhaft 2 bis 3, insbesondere 2 bis 2,5 mol Olefin pro Mol Benzylhalogenid I verwendet werden. Niedrigere, z.B. äquimolare Olefinmengen sind möglich, aber nicht zweckmäßig, da der bei der Reaktion entstehende Halogenwasserstoff eventuell vom Olefin gebunden und dadurch verbraucht werden kann. Höhere Überschüsse an Olefin sind dagegen möglich, bringen aber in der Regel keine Vorteile.

Die Umsetzung wird erfindungsgemäß in Gegenwart katalytischer Mengen an Titantetrachlorid durchgeführt, da dieser Katalysator die Ringschlußreaktion zum Indan besonders begünstigt. Die Katalysatormenge liege zweckmäßigerweise bei 0,002 bis 0,2, insbesondere 0,004 bis 0,04, vorzugsweise 0,01 bis 0,03 mol, bezogen auf 1 mol Benzylhalogenid I. Bei geringeren und höheren Katalysatormengen nimmt im allgemeinen die Bildung von Nebenprodukten zu.

Die Umsetzung kann in Gegenwart oder in Abwesenheit eines Lösungsmittels durchgeführt werden. Als Lösungsmittel können die für Friedel-Crafts--Reaktionen üblichen Lösungsmittel wie aromatische oder aliphatische Kohlenwasserstoffe, Ether oder halogenierte Kohlenwasserstoffe, z.B. Benzol, Toluol, Hexan, Diethylether, Dichlormethan, Chloroform von Lösungsmitteln nicht nötig, was das erfindungsgemäße Verfahren besonders auszeichnet.

Die Reaktionstemperatur richtet sich nach der Reaktivität der Einsatzstoffe. Sie liegt in der Regel bei ca. -20 bis +40 °C. Tiefere Temperaturen sind möglich, aber im allgemeinen nicht nötig. Die Durchführung kann in an sich bekannter Weise bei Überdruck, Unterdruck oder vorteilhaft bei Atmosphärendruck, diskontinuierlich oder kontinuierlich nach den dafür üblichen Techniken vorgenommen werden.

Beispielsweise kann man das Olefin bei der Reaktionstemperatur zusammen mit Titantetrachlorid vorlegen und das Benzylhalogenid I hinzufügen. Das erhaltene Umsetzungsgemisch wird in an sich üblicher Weise aufgearbeitet, indem man z.B. den Katalysator durch Zugabe von Wasser zerstört, überschüssiges Olefin und flüchtige Nebenprodukte abdestilliert und das im Rückstand enthaltene Indan z.B. durch Destillation oder Umkristallisieren reinigt oder direkt weiterverwendet.

Nach dem erfindungsgemäßen Verfahren können gegebenenfalls substituierte Indane, insbesondere polyalkylsubstituierte Indane, auf technisch einfache Weise in guten Ausbeuten erhalten werden. Die Produkte, die als Zwischenprodukte z.B. für die Herstellung von Riechstoffen von Interesse sind, fallen häufig so rein an, daß sie direkt ohne destillative Reinigung weiterverarbeitet werden können. Die Herstellung von bicyclischen aromatischen Moschus-Riechstoffen wie Galaxolid aus den erfindungsgemäß erhaltenen Indanen ist z.B. in EP 89207 oder den US-Patenten 4 265 818 und 4 315 951 beschrieben.

Beispiel 1

Herstellung 1,1,2,3,3-Pentamethylindan

Bei -5 °C wurden 1750 g (25 mol) 2-Methylbut-2-en zusammen mit 32 g (0,17 mol) Titantetrachlorid vorgelegt und innerhalb von 3 Stunden mit 1547 g (10 mol) 2-Chlor-2-phenylpropan (Cumylchlorid) versetzt, wobei die Temperatur nicht über +10 °C anstieg. Anschließend wurde 1 h bei Raumtemperatur nachgerührt und das Reaktionsgemisch wie üblich aufgearbeitet, indem man 12,3 g (0,68 mol) Wasser zusetzte, bei Raumtemperatur rührte und nach Phasentrennung das überschüssige Olefin und das als Nebenprodukt entstandene tert.-Amylchlorid aus der organischen Phase abdestillierte.

Es verblieben 1851 g Rückstand, der gemäß gaschromatographischer Analyse 94 Gew.-% 1,1,2,3,3-Pentamethylindan, entsprechend einer Ausbeute von 92,5 %, bezogen auf Cumylchlorid, enthielt und direkt für Folgereaktionen weiterverwendet werden konnte.

Bei einer entsprechend Beispiel 1 jedoch in Gegenwart von 8 g (0,042 mol) Titantetrachlorid durchgeführten Umsetzung fiel das Produkt nach destillativer Reinigung in einer Ausbeute von 80,3 %, bezogen auf Cumylchlorid, an.

Vergleichsbeispiel 1

a) 87,5 (1,25 mol) 2-Methylbut-2-en wurden zusammen mit 1,19 g (8,5 mmol) wasserfreiem Zinkchlorid in 0,38 g (5,1 mmol) Diethylether bei 0° C vorgelegt und bei dieser Temperatur innerhalb von 1 Stunde mit 78 g (0,5 mol) Cumylchlorid versetzt. Anschließend wurde 1 Stunde bei Raumtemperatur nachgerührt.

Nach üblicher Aufarbeitung und Abdestillieren von Leichtsiedern wurden 75,7 g Rückstand erhalten, der gemäß gaschromatographischer Analyse 11 Gew.-% 1,1,2,3,3-Pentamethylindan, entsprechend einer Ausbeute von 8,9 %, bezogen auf Cumylchlorid, enthielt.

b) Nach Umsetzung analog a), jedoch in Gegenwart von 132 ml Methylenchlorid, wurden 50,9 % 1,1,2,3,3-Pentamethylindan, bezogen auf Cumylchlorid, erhalten (gaschromatographisch ermittelt).

Beispiel 2

Herstellung von 1,1,2,2,3,3-Hexamethylindan

110 g (1,31 mol) Tetramethylethylen und 82,8 g (0,535 mol) Cumylchlorid wurden in 1,6 1 Methylenchlorid gelöst und auf -75° C gekühlt. Innerhalb von 10 min wurden 10 ml Titantetrachlorid zugegeben und das Reaktionsgemisch 1 h bei -75° C gerührt. Die Lösung wurde anschließend auf 900 ml verdünnte Salzsäure gegossen, die organische Phase abgetrennt und getrocknet. Nach Abfiltrieren des Trockenmittels wurde der Rückstand nach Abtrennen von Leichtsiedern destilliert.
Ausbeute: 78,2 g (≙ 72 % der Theorie) 1,1,2,2,3,3-Hexamethylindan,
Sdp. 52 - 55° C/0,5 mbar. 'H-NMR ($CCL_4$): $\delta$ = 0,85 (s, 6 H), 1,17 (12 H), 7,00 (s, 4 H).

## Ansprüche

1. Verfahren zur Herstellung von Indanen durch Umsetzung von gegebenenfalls substituierten Benzylhalogeniden mit Olfinen in Gegenwart einer Lewis-Säure als Katalysator, dadurch gekennzeichnet, daß man Benzylhalogenide der Formel I

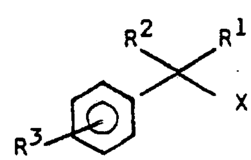

I,

in der die Reste $R^1$, $R^2$ und $R^3$ Wasserstoff oder einen Alkyl-, Alkoxy-, Cycloalkyl-, Aryl-, Aryloxy- oder Aralkylrest bedeuten, $R^3$ darüber hinaus für Halogen stehen kann oder ein ortho-kondensiertes Ringsystem bildet und X Halogen bedeutet, mit Olefinen der Formel II

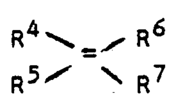

II,

in der die Reste $R^4$ bis $R^7$ Wasserstoff oder einen in alpha-Stellung unverzweigten Alkylrest darstellen,

in Gegenwart Katalytischer Mengen an Titantetrachlorid umsetzt.

2. Verfahren nach Anspruch 1, dadurch gekennzeichnet, daß man Benzylchloride oder -bromide umsetzt.

3. Verfahren nach den Ansprüchen 1 und 2, dadurch gekennzeichnet, daß man 2 bis 3 mol Olefin II pro Mol Benzylhalogenid I verwendent.

4. Verfahren nach den Ansprüchen 1 bis 3, dadurch gekennzeichnet, daß man 0,004 bis 0,04 mol Titantetrachlorid pro Mol Benzylhalogenid I verwendet.

5. Verfahren nach den Ansprüchen 1 bis 3, dadurch gekennzeichnet, daß man 0,01 bis 0,03 mol Titantetrachlorid pro Mol Benzylhalogenid I verwendet.

6. Verfahren nach den Ansprüchen 1 bis 5, dadurch gekennzeichnet, daß man die Umsetzung in Abwesenheit eines Lösungsmittels vornimmt.

7. Verfahren nach den Ansprüchen 1 bis 6, dadurch gekennzeichnet, daß man die Umsetzung bei Temperaturen von -20 bis +40 °C vornimmt.

## Claims

1. A process for preparing an indane by reacting a substituted or unsubstituted benzyl halide with an olefin in the presence of a Lewis acid as catalyst, which comprises reacting a benzyl halide of the formula I

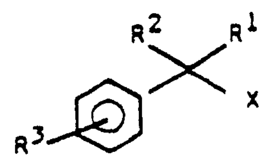

I

where $R^1$, $R^2$ and $R^3$ are each hydrogen, alkyl, alkoxy, cycloalkyl, aryl, aryloxy or aralkyl, $R^3$ may in addition be halogen or form an ortho-fused ring system, and x is halogen, with an olefin of the formula II

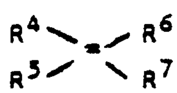

II

where $R^4$, $R^5$, $R^6$ and $R^7$ are each hydrogen or alkyl which is unbranched in the alpha position, in the presence of a catalytic amount of titanium tetrachloride.

2. A process as claimed in claim 1, wherein a benzyl chloride or bromide is reacted.

3. A process as claimed in either of claims 1 and 2, wherein from 2 to 3 moles of olefin II are used per mole of benzyl halide I.

4. A process as claimed in any of claims 1 to 3, wherein from 0.004 to 0.04 mol of titanium tetrachloride is used per mole of benzyl halide I.

5. A process as claimed in any of claims 1 to 3, wherein from 0.01 to 0.03 mol of titanium tetrachloride is used per mole of benzyl halide I.

6. A process as claimed in any of claims 1 to 5, wherein the reaction is carried out in the absence of a solvent.

7. A process as claimed in any of claims 1 to 6, wherein the reaction is carried out at from -20 to +40° C.

## Revendications

1. Procédé de préparation d'indanes par réaction d'halogénures de benzyle éventuellement substitues avec des oléfines en présence d'un acide de Lewis servant de atalyseur, caractérisé en ce qu'on fait réagir, en présence de quantités catalytiques de tétrachlorure de titane, des halogénures de benzyle de formule I

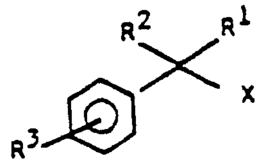

I,

dans laquelle les restes $R^1$, $R^2$ et $R^3$ représentent chacun un atome d'hydrogène ou un groupement alkyle, alcoxy, cycloalkyle, aryle, aryloxy ou aralkyle, $R^3$ peut en outre être mis pour un atome d'halogène ou forme un système cyclique orthocondensé et X représente un atome d'halogène, avec des oléfines de formule II

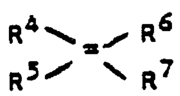

II,

dans laquelle les restes $R^4$ à $R^7$ représentent chacun un atome d'hydrogène ou un groupement alkyle non ramifié en position α.

2. Procédé selon la revendication 1, caractérisé en ce qu'on fait réagir des chlorures ou des bromures de benzyle.

3. Procédé selon la revendication 1 ou 2, caractérisé en ce qu'on utilise 2 à 3 mol d'oléfine II par moi d'halogénure de benzyle I.

4. Procédé selon l'une quelconque des revendications 1 à 3, caractérisé en ce qu'on utilise de 0,004 à 0,04 mol de tétrachlorure de titane par mol d'halogénure de henzyle I.

5. Procédé selon l'une quelconque des revendications 1 à 3, caractérisé en ce qu'on utilise de 0,01 à 0,03 mol de tétrachlorure de titane par mol d'halogénure de benzyle I.

6. Procédé selon l'une quelconque des revendications 1 à 5, caractérisé en ce qu'on procède à la réaction en l'absence d'un solvant.

7. Procédé selon l'une quelconque des revendications 1 à 6, caractérisé en ce qu'on procède à la réaction à des températures de -20 à +40° C.